# EUROPEAN PATENT SPECIFICATION

(11) **EP 3 269 358 B1**
(45) Date of publication and mention of the grant of the patent: **05.08.2020**
(21) Application number: 17165777.8
(22) Date of filing: 11.11.2010
(51) Int. Cl.: A61K 9/00, A61Q 3/00, A61K 8/36, A61K 31/19, A61K 47/12, A61K 47/14, A61K 8/365, A61Q 17/00, A61M 35/00

(54) **COMPOSITION FOR TOPICAL APPLICATION, USES THEREOF, APPLICATOR DEVICE AND KIT OF PARTS**
ZUSAMMENSETZUNG ZUR TOPISCHEN ANWENDUNG, IHRE VERWENDUNG, APPLIKATOR UND TEILSATZ
COMPOSITION POUR APPLICATION TOPIQUE, SES UTILISATIONS, DISPOSITIF D'APPLICATION ET NÉCESSAIRE ASSOCIÉ

(30) Priority: 11.11.2009 NL 2003786
(43) Date of publication of application: 17.01.2018
(62) Divisional of application: 10796170.8
(73) Proprietor: YouMedical B.V., 1059 AT Amsterdam (NL)
(72) Inventor: STAL, Robert, Sebastian, 1018 HA Amsterdam (NL)
(74) Representative: Brann AB

(56) References cited:
- WO-A2-2008/128627
- FR-A1- 2 394 290
- US-A1- 2008 112 908
- US-B1- 6 740 326

## Description

### FIELD OF THE INVENTION

The invention relates to a composition for topical application. The invention further relates to an applicator device and further discloses a kit of parts comprising such a composition.

### BACKGROUND OF THE INVENTION

Onychomycosis is a wide-spread microbiological infection of the keratin of the nail. The fungi are able to enter the nail through microscopic cracks in the nail. Once in the nail the fungus uses the macroscopic molecules in the nail to feed itself and alter the environment to its own advantage. One of these alterations includes raising the pH of the nail to an alkaline level. This creates a favourable environment for the fungus in which it can multiply sexually instead of asexually.

US2008/0112908 discloses an anti-fungal nail lacquer composition comprising a film forming agent, a solvent, pigment and a glacial acetic acid.

### OBJECT AND SUMMARY OF THE INVENTION

It is an object of the invention to provide a safe and effective treatment for onychomycosis.

The invention relates to a composition according to claim 1 for topical application, comprising at least one physiologically acceptable acid capable of reducing the pH of the skin and/or nails of a treated person to the range of 1.5-4.5, and a physiologically acceptable carrier. Bringing the pH into the range of 1.5-4.5 hampers the development of onychomycosis. The acid can be incorporated in for instance a cream of lotion. The physiologically acceptable carrier should be selected for not neutralising the effect of the acid.

The physiologically acceptable acid is a carboxylic acid. Carboxylic acids are capable of achieving the desired pH drop on the infected skin and/or nails, and are typically well tolerated by the treated person. Preferably, the carboxylic acid is an alpha- hydroxy carboxylic acid.

Most preferably, the physiologically acceptable acid is a C1-C6 carboxylic acid.

In a preferred embodiment, the carboxylic acid is elected from the group consisting of lactic acid, malic acid, tartaric acid, citric acid, acetic acid, proprionic acid, isoproprionic acid, oxalic acid, glutaric acid, adipic acid, glycolic acid and mandelic acid.

Most preferably, the carboxylic acid is lactic acid. Lactic acid gives the desired result against onychomycosis and is well tolerated by persons.

Another preferred carboxylic acid is acetic acid. Acetic acid gives good results against onchyomycosis, is well tolerated. As acetic acid is a relatively cheap compound, in particular compared to lactic acid, acetic acid is a good alternative for lactic acid, and may also be used in an acetic acid/lactic acid combination in order to diminish the amount of lactic acid used while retaining the desired skin or nail acidifying effect.

It is preferred if the carboxylic acid is present in a quantity of at least 1% by weight. In this quantity, the development onychomycosis is effectively hampered.

Composition according to any of the preceding claims, wherein the composition comprises at least 10% by weight of lactic acid. In this quantity, the development onychomycosis is severely hindered and in some cases even stopped.

The composition also comprises at least one C1-C4 alkyl ester of the carboxylic acid in a molecular ratio free acid to the ester of at least 1:20, most preferably at least 1:10. Such C1-C4 alkyl esters are excellent carriers for the acid.

The composition comprises lactic acid as the physiologically acceptable acid, and lactic acid ethyl ester as a physiologically acceptable carrier.

Also disclosed, but not claimed, is that the composition comprises acetic acid as the physiologically acceptable acid, and lactic acid ethyl ester as a physiologically acceptable carrier.

It is also possible to obtain a satisfactory composition by mixing lactic acid and acetic acid as physiologically acceptable acids, using lactic acid ethyl ester as a physiologically acceptable carrier.

The invention further relates to the use of a composition according to the invention for the preparation of a product for the treatment of onychomycosis.

The invention relates to a device, comprising a container comprising a composition according to the invention, and an applicator connected to the container, wherein the applicator is adapted to apply the composition from the container to a part of human skin and/or nails to be treated.

The invention provides a composition for topical application, including an effective amount of lactic acid, and/or a physiologically acceptable ester or salt thereof. The combination of lactic acid, or a similar acid, or a derivative thereof and at least one physiologically acceptable carrier to aid penetration into the nail were shown to yield an improved effect in the treatment of skin and nail conditions, in particular the treatment of onychomycosis. It is postulated that nail conditions benefit from the ability of lactic acid in controlling the pH of the nail, an essential part of this formula to combat nail fungus. This is enhanced by presence of the C1-C4 alkyl ester of a carboxylic acid as a carrier and stabilizer, which improves the penetration of lactic acid or its derivatives into the skin or nail.

The lactic acid is preferably mixed homogeneously with the C1-C4 alkyl ester of a carboxylic acid. Most preferably, the lactic acid is dissolved in the C1-C4 alkyl ester of a carboxylic acid. The C1-C4 alkyl ester of a carboxylic acid is preferably present as a major component of the composition, in a quantity of at least 50% by weight of the composition, preferably at least 90%.

The composition may include additional suitable components, for instance fragrances, emulsifiers, detergents, antioxidants and preservatives, and other ingredients commonly used in pharmaceutical and cosmetic formulations.

Preferably, the composition is essentially free of water, which increases the stability of the composition over time.

Preferably, the composition is formulated as a fluid composition such as a cream, or more preferably as a liquid composition such as a tonic, which is relatively easy to apply to the human skin and/or nails.

The C1-C4 esters include methyl, ethyl, n-propyl, isopropyl, n-butyl, isobutyl, sec-butyl and tert-butyl esters. Ethyl esters are preferred. In case the carboxylic acid contains multiple carboxyl-groups, at least one of the carboxyl groups is a C1-C4 ester. In case at least two carboxyl groups are esterified, the C1-C4 esters of these groups may be the same or different. The C1-C4 alkyl ester is derived from a physiologically acceptable alpha-hydroxy carboxylic acid.

The C1-C4 alkyl ester is derived from a physiologically acceptable carboxylic acid selected from lactic acid. The C1-C4 esters of this acid are particularly effective. The ethyl esters of these compounds are preferred. C1-C4 alkyl esters of lactic acid was shown to be the most versatile of these compounds, and is therefore the most preferred.

In a preferred embodiment, the C1-C4 alkyl ester is lactic acid ethyl ester. This compound showed the best results.

Instead of lactic acid as the free acid, also lactic acid salt derivatives or esters may be used. Good results are obtained when the composition comprises at least one lactic acid derivative selected from the group consisting of sodium salicylate, potassium salicylate, calcium salicylate, magnesium salicylate, bismuth subsalicylate, monoethylammonium (MEA) salicylate, triethylammonium (TEA) salicylate, sulfolactic acid, ethyl salicylate, butyloctyl salicylate, C12-15 Alkyl salicylate, capryolyl lactic acid, hexyldecyl salicylate, isocetyl salicylate, isodecyl salicylate, ethylhexyl salicylate, methyl salicylate, myristyl salicylate, and tridecyl salicylate. Instead of lactic acid, an equivalent dose of a derivative having the same effect may be used. For salicylate salts, this is usually an equimolar amount. A composition may comprise a mix of lactic acid and/or one or more lactic acid derivatives.

In a preferred embodiment, the composition comprises at least 0.1% by weight of lactic acid or a physiologically acceptable ester or salt thereof, preferably between 0.5 and 30% by weight. Such amounts have an advantageous effect on various skin and/or nail conditions, in particular onychomycosis. The amount may be optimized for a specific skin and/or nail condition.

The composition also comprises the free acid or salt derived from the carboxylic acid used in the C1-C4 alkyl ester in a molecular ratio of at least 1:20 with respect to the C1-C4 alkyl ester most preferably at least 1:10. Thus, a more stable composition is achieved. The availability of the carboxylic acid prevents the reversal of the esterification reaction in the presence of water, resulting in the free carboxylic acid and alcohol. For instance, a composition according to the invention, based on lactic acid ethyl ester, could be stabilized by adding lactic acid in a ratio of at least 1:20, most preferably at least 1:10.

Preferably, the composition also comprises a nail-penetrating agent. A nail penetrating agent allows the acid and optional other active ingredients to permeate into the nail, which was found to lead to a better inhibition of the growth of nail infections, in particular fungal nail infections.

Preferred nail-penetrating agents are selected from the group consisting of urea, dimethyl isosorbide and ethyl lactate, C1-C4 alkyl ester of the carboxylic acid, preferably ethyl lactate or ethyl acetate. Mixtures of various nail penetrating agents may be used advantageously.

The invention relates to the use of a composition according to the invention for the preparation of a product, for instance a medicament, for the treatment of skin and nail conditions. Skin conditions the composition according to the invention may be used against conditions including onychomycosis and other microbiological infections. Typically, the amount of lactic acid or derivatives thereof are within 0.1-30% by weight.

The invention also relates to a device, comprising a container comprising a composition according to the invention, and an applicator connected to the container, wherein the applicator is adapted to apply the composition from the container to a part of human skin to be treated. Thus, the composition according to the invention is easy to apply and store.

### BRIEF DESCRIPTION OF THE DRAWINGS

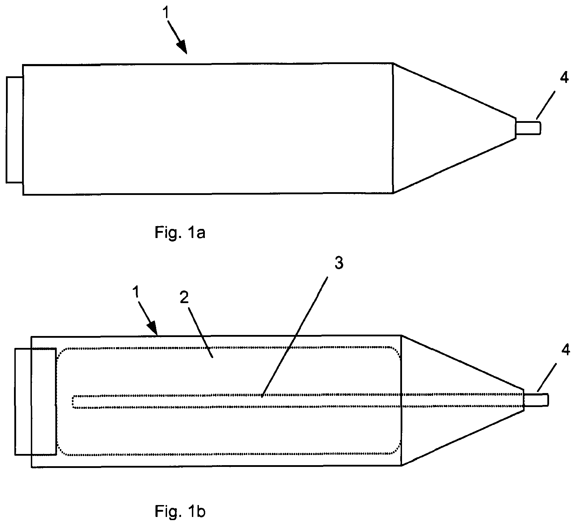

Figure 1 shows an applicator for applying a composition according to the invention.

### DESCRIPTION OF PREFERRED EMBODIMENTS

The invention will now be further elucidated by the following non-limiting examples.

### Applicator device

Figure 1 shows an application device, somewhat resembling a felt-tipped type marker. The device comprises a reservoir containing a composition according to the invention. The device is pencil-shaped, and suitable to be hand-held. The reservoir is provided with an absorbing element made of a liquid-absorbing material capable of capillary action. This absorbing element dips into the liquid composition and extends from the inner part reservoir to the outside of the reservoir. The liquid contained in the reservoir within the marker and would be applied via a tip connecting to or made out of the distal end of the absorbing element extending out of the reservoir. Due to capillary action, the tip remains moist with the liquid product. For such an applicator device, the viscosity of the liquid composition will have to be sufficiently low. The device may be provided with a cap to prevent volatile solvents of the composition to evaporate and dry the tip out. The moist tip is contacted with the skin part to be treated, preferably while applying some pressure, in order to apply the composition from the reservoir.

The reservoir may be filled with different preferred embodiments according to the invention, depending on the envisaged skin condition to be treated. For all of these applications it was shown that the combination of lactic acid dissolved in or mixed with at least one physiologically acceptable C1-C4 alkyl ester of a carboxylic acid as a carrier yielded a better absorbance of lactic acid into the skin than a comparable aqueous composition with the same lactic acid concentration.

All components are commercially available; the compositions were prepared using well-known mixing and blending techniques. pH measurements on the skin can be determined using commercially available pH meters, for instance the pH meter '1140' from Mettler Toledo.

### Example 1: onychomycosis

An effective composition for treatment of onychomycosis was prepared by well know mixing techniques (percentages by weight).:

| | |
|---|---|
| lactic acid | 5% |
| lactic acid ethylester | 95% |

The resulting liquid is applied as a tonic, and showed a changed environment of the nail to the fungus disadvantage. The pH on the skin of treated persons temporarily dropped to a value in the range of 2-4. Such low pH environments appear to hamper the development of onychomycosis. Over time this effectively treated the nail of nail fungus.

### Example 2: onychomycosis

An effective composition for treatment of onychomycosis was prepared by well know mixing techniques (percentages by weight):

| | |
|---|---|
| Melaleuca Alternifolia | 1% |
| Lavendula Officinalis | 2% |
| Callitris Intratropica | 2% |
| lactic acid | 2% |
| lactic acid ethyl ester | 93% |

The resulting liquid is applied as an tonic, and showed a changed the environment of the nail to the fungus disadvantage, showing a temporary pH drop of the skin to approximately 3-4. Overtime this effectively treated the nail of nail fungus. This example is cosmetically more attractive than example 1.

### Example 3: Microbial infections of the skin.

An effective composition against warts was prepared by well know mixing techniques (percentages by weight):

| | |
|---|---|
| lactic acid | 20% |
| lactic acid ethyl ester | 80% |

Upon application to the skin, the pH of the skin dropped to approximately 2.

Various types of microbial infections were successfully treated using this composition, resulting in complete or partial removal of the infection after multiple treatments.

### Reference Example 4: onychomycosis

An effective composition for treatment of onychomycosis was prepared by well know mixing techniques (percentages by weight).:

| | |
|---|---|
| acetic acid | 5% |
| ethyl acid ethyl ester | 95% |

The resulting liquid is applied as a tonic, and showed a changed environment of the nail to the fungus disadvantage. The pH on the nail of treated persons temporarily dropped to a value in the range of 2-4. Such low pH environments appear to hamper the development of onychomycosis. Over time this effectively treated the nail of nail fungus. The same composition using a mixture of 1% lactic acid and 4% acetic acid instead of 5% acetic acid also yielded satisfactory results against onchyomycosis.

### Example 5: onychomycosis

An effective composition for treatment of onychomycosis was prepared by well know mixing techniques (percentages by weight):

| | |
|---|---|
| Lactic acid | 15% |
| Urea | 5% |
| Lactic acid ethyl ester | 80% |

The resulting liquid is applied as an tonic, and showed a changed the environment of the nail to the fungus disadvantage, showing a temporary pH drop of the nail to approximately 3-4. Over time this effectively treated the nail of nail fungus. This example is cosmetically more attractive than example 1.

### Reference Example 6: Microbial infections of the nail.

An effective composition against nail microbial infections was prepared by well know mixing techniques (percentages by weight):

| | |
|---|---|
| acetic acid | 15% |
| dimethyl isosorbide | 10% |
| acetic acid ethyl ester | 75% |

Upon application to the skin, the pH of the nail dropped to approximately 2.5. Dimethyl isosorbide acts as a nail-penetrating agent, enhancing the penetration by acetic acid. Various types of microbial infections were successfully treated using this composition, resulting in complete or partial removal of the infection after multiple treatments.

The compositions shown in the examples above may be used as such, or may be processed further to a final product, for instance by diluting with water or other solvents, or incorporation into a cream or lotion.

## Claims

1. Composition for the treatment of fungal infections, comprising:
• at least one physiologically acceptable carboxylic acid capable of reducing the pH in keratinous tissue, in particular nails, of a treated person below 4.5, preferably in the range of 1.5-4.5; and
• a physiologically acceptable carrier selected from C1-C4 alkyl ester derived from a physiolocially acceptable alpha-hydroxy carboxylic acid;
wherein the C1-C4 alkyl ester is a C1-C4 alkyl ester of lactic acid; and wherein the composition also comprises the free acid or salt derived from the carboxylic acid used in the C1-C4 alkyl ester in a molecular ratio of at least 1:20 with respect to the C1-C4 alkyl ester.

2. The composition according to claim 1 wherein the C1-C4 alkyl ester derived from a physiolocially acceptable alpha-hydroxy carboxylic acid is lactic acid ethyl ester.

3. The composition according to any one of claim 1 or 2 wherein the carboxylic acid is present in a quantity of at least 1% by weight.

4. The composition according to claim 2 wherein the C1-C4 alkyl ester derived from a physiolocially acceptable alpha-hydroxy carboxylic acid is lactic acid ethyl ester and wherein the composition is stabilized by addition of lactic acid in a ratio of at least 1:10 to the lactic acid ethyl ester.

5. The composition according to claim 1 wherein the at least one physiologically acceptable carboxylic acid is selected from malic acid, tartaric acid, citric acid, acetic acid, proprionic acid, isoproprionic acid, oxalic acid, glutaric acid, adipic acid, glycolic acid and mandelic acid.

6. The composition according to any one of claims 1 to 5 for use in the treatment of onychomosis.

7. An application device comprising a reservoir containing the composition according to any one of claim 1 to 6 wherein the device is pencil-shaped and suitable to be hand-held, wherein the reservoir is provided with an absorbing element made of a liquid-absorbing material capable of capillary action, wherein the absorbing element dips into the liquid composition and extends from the inner part reservoir to the outside of the reservoir and wherein the liquid composition contained in the reservoir may be applied via tip connected to or made out of the distal end of the absorbing element extending out of the reservoir.

## Patentansprüche

1. Zusammensetzung zur Behandlung von Pilzinfektionen, umfassend
- mindestens eine physiologisch unbedenkliche Carbonsäure, die geeignet ist, den pH in keratinösem Gewebe, insbesondere Nägeln, einer behandelten Person unter 4,5, bevorzugt in dem Bereich von 1,5 - 4,5, zu senken; und
- einen physiologisch unbedenklichen Träger, ausgewählt aus C1-C4-Alkylester, der von einer physiologisch unbedenklichen Alpha-Hydroxycarbonsäure abgeleitet ist;
wobei der C1-C4-Alkylester ein C1-C4-Alkylester von Milchsäure ist; und wobei die Zusammensetzung auch die freie Säure oder das Salz umfasst, die oder das von der Carbonsäure abgeleitet ist, die in dem C1-C4-Alkylester in einem Molekularverhältnis von mindestens 1:20 in Bezug auf den C1-C4-Alkylester verwendet wird.

2. Zusammensetzung nach Anspruch 1, wobei der C1-C4-Alkylester, der von einer physiologisch unbedenklichen Alpha-Hydroxycarbonsäure abgeleitet ist, Milchsäureethylester ist.

3. Zusammensetzung nach einem von Anspruch 1 oder 2, wobei die Carbonsäure in einer Menge von mindestens 1 Gew.-% vorhanden ist.

4. Zusammensetzung nach Anspruch 2, wobei der C1-C4-Alkylester, der von einer physiologisch unbedenklichen Alpha-Hydroxycarbonsäure abgeleitet ist, Milchsäureethylester ist und wobei die Zusammensetzung durch Zugabe von Milchsäure in einem Verhältnis von mindestens 1:10 zu dem Milchsäureethylester stabilisiert ist.

5. Zusammensetzung nach Anspruch 1, wobei die mindestens eine physiologisch unbedenkliche Carbonsäure aus Apfelsäure, Weinsäure, Zitronensäure, Essigsäure, Propansäure, Isopropansäure, Oxalsäure, Glutarsäure, Adipinsäure, Glycolsäure und Mandelsäure ausgewählt ist.

6. Zusammensetzung nach einem der Ansprüche 1 bis 5 zur Verwendung bei der Behandlung von Onychomykose.

7. Anwendungsvorrichtung, die einen Behälter umfasst, der die Zusammensetzung nach einem von Anspruch 1 bis 6 enthält, wobei die Vorrichtung stiftförmig ist und handgehalten werden kann, wobei der Behälter mit einem absorbierenden Element bereitgestellt ist, das aus einem flüssigkeitsabsorbierenden Material hergestellt ist, das zu Kapillarwirkung geeignet ist, wobei das absorbierende Element in die flüssige Zusammensetzung taucht und sich von dem inneren Teil des Behälters zu der Außenseite des Behälters erstreckt und wobei die flüssige Zusammensetzung, die in dem Behälter enthalten ist, über eine Spitze, die mit dem distalen Ende des absorbierenden Elements, das sich aus dem Behälter erstreckt, verbunden oder daraus hergestellt ist, aufgebracht werden kann.

## Revendications

1. Composition destinée au traitement des infections fongiques, comprenant :
• au moins un acide carboxylique physiologiquement acceptable capable d'abaisser le pH dans le tissu kératinique, en particulier les ongles, d'une personne traitée en dessous de 4,5, de préférence dans la plage de 1,5 à 4,5 ; et
• un véhicule physiologiquement acceptable choisi parmi un ester d'alkyle en C1-C4 dérivé d'un acide alpha-hydroxy carboxylique physiologiquement acceptable ;
dans laquelle l'ester d'alkyle en C1-C4 est un ester d'alkyle en C1-C4 d'acide lactique; et dans laquelle la composition comprend également l'acide libre ou un sel dérivé de l'acide carboxylique utilisé dans l'ester d'alkyle en C1-C4 en un rapport moléculaire d'au moins 1:20 par rapport à l'ester d'alkyle en C1-C4.

2. Composition selon la revendication 1 dans laquelle l'ester d'alkyle en C1-C4 dérivé d'un acide alpha-hydroxy carboxylique physiologiquement acceptable est l'ester éthylique de l'acide lactique.

3. Composition selon l'une quelconque de la revendication 1 ou 2 dans laquelle l'acide carboxylique est présent dans une quantité d'au moins 1 % en poids.

4. Composition selon la revendication 2 dans laquelle l'ester d'alkyle en C1-C4 dérivé d'un acide alpha-hydroxy carboxylique physiologiquement acceptable est l'ester éthylique de l'acide lactique et dans laquelle la composition est stabilisée par addition d'acide lactique en un rapport d'au moins 1:10 à l'ester éthylique de l'acide lactique.

5. Composition selon la revendication 1 dans laquelle l'au moins un acide carboxylique physiologiquement acceptable est choisi parmi l'acide malique, l'acide tartrique, l'acide citrique, l'acide acétique, l'acide proprionique, l'acide isoproprionique, l'acide oxalique, l'acide glutarique, l'acide adipique, l'acide glycolique et l'acide mandélique.

6. Composition selon l'une quelconque des revendications 1 à 5 pour une utilisation dans le traitement de l'onychomycose.

7. Dispositif d'application comprenant un réservoir contenant la composition selon l'une quelconque de la revendication 1 à 6 dans lequel le dispositif est en forme de crayon et approprié pour être de poche, dans lequel le réservoir est doté d'un élément absorbant fait d'un matériau absorbant les liquides capable d'action capillaire, dans lequel l'élément absorbant plonge dans la composition liquide et s'étend de la partie interne du réservoir à l'extérieur du réservoir et dans lequel la composition liquide contenue dans le réservoir peut être appliquée via un embout connecté à ou fait de l'extrémité distale de l'élément absorbant s'étendant hors du réservoir.
